# EUROPÄISCHE PATENTSCHRIFT

(11) **EP 0 554 660 B1**
(45) Veröffentlichungstag und Bekanntmachung des Hinweises auf die Patenterteilung: **23.10.1996**
(21) Anmeldenummer: 93100118.4
(22) Anmeldetag: 07.01.1993
(51) Int. Cl.: C07C 57/04, C07C 51/44, C07C 51/487

(54) **Verfahren zur Reinigung von Rohsäuren alpha-beta-monoethylenisch ungesättigter Carbonsäuren**
Process for the purification of alpha-beta-monoethylenic crude acids and of insaturated carboxylic acids
Procédé de purification d'acides bruts alpha-bêta-monoéthyléniques et d'acides carboxyliques insaturés

(30) Priorität: 23.01.1992 DE 4201697
(43) Veröffentlichungstag der Anmeldung: 11.08.1993
(73) Patentinhaber: BASF Aktiengesellschaft, 67063 Ludwigshafen (DE)
(72) Erfinder: Duembgen, Gerd, Dr., 6200 Wiesbaden (DE); Fiedler, Eckhard, Dr., W-6108 Weiterstadt (DE); Hammon, Ulrich, Dr., W-6800 Mannheim 1 (DE); Thiessen, Fritz, Dr., W-6700 Ludwigshafen (DE); Vogel, Herbert, Dr., W-6700 Ludwigshafen (DE)

(56) Entgegenhaltungen:
- EP-A- 0 102 642
- EP-A- 0 169 254
- DE-A- 2 136 396
- DE-A- 2 207 184
- DE-A- 4 023 239
- JP-A-55 129 239

## Beschreibung

Die vorliegende Erfindung betrifft ein Verfahren zur Reinigung von Rohsäuren α,β-monoethylenisch ungesättigter Carbonsäuren, die nach dem Verfahren der katalytischen Gasphasenoxidation erzeugt wurden.

α,β-monoethylenisch ungesättigte Carbonsäuren wie Acryl- und Methacrylsäure, entweder für sich oder in Form ihrer Ester, sind insbesondere zur Herstellung von Polymerisaten für die verschiedensten Anwendungsgebiete, z.B. Verwendung als Klebstoffe, von Bedeutung.

Unter anderem sind α,β-monoethylenisch ungesättigte Carbonsäuren durch katalytische Gasphasenoxidation von Alkanen, Alkanolen, Alkanalen, Alkenen oder Alkenalen erhältlich. Beispielsweise kann Acrylsäure durch katalytische Gasphasenoxidation von Propan, Propen oder Acrolein hergestellt werden und Methacrylsäure ist z.B. durch katalytische Gasphasenoxidation von tert.-Butanol, iso-Buten, iso-Butan, iso-Butyraldehyd oder Methacrolein erhältlich. Dabei werden diese Ausgangsgase, in der Regel mit inerten Gasen wie Stickstoff, CO₂ und/oder Wasserdampf verdünnt, im Gemisch mit Sauerstoff bei erhöhten Temperaturen sowie gegebenenfalls erhöhtem Druck über übergangsmetallische Mischoxidkatalysatoren geleitet, oxidativ in die entsprechende α,β-monoethylenisch ungesättigte Säure umgewandelt und durch die Aufnahme in ein geeignetes Absorptionsmittel (z.B. Wasser oder ein Gemisch aus 70 bis 75 Gew.-% Diphenylether und 25 bis 30 Gew.-% Diphenyl) aus dem Produktgasstrom abgetrennt (vgl. z.B. EP-A 297 445 und DE-PS 2 136 396).

Nach Entfernung des Absorptionsmittels (und gegebenenfalls zuvor erfolgter Desorption von eine geringe Absorptionsmittellöslichkeit aufweisenden Verunreinigungen durch Abstreifen, z.B. mit Luft) über extraktive und/oder destillative Trennverfahren (z.B. Entfernung des Absorptionsmittels Wasser durch Destillation, azeotrope Destillation oder extraktive Abtrennung der Säure aus der wäßrigen Lösung und anschließende destillative Entfernung des Extraktionsmittels) wird eine Säure erhalten, die hier als Rohsäure bezeichnet wird.

Aufgrund zahlreicher im Verlauf der katalytischen Gasphasenoxidation erfolgender Parallel- und Folgereaktionen bildet die Rohsäure kein reines Produkt. Vielmehr enthält sie ein Spektrum verschiedener Verunreinigungen (in der Regel in der Größenordnung von ≦ 2 Gew.-%, vgl. EP-B 169 254), das hauptsächlich aus mit den Ausgangsverbindungen der katalytischen Gasphasenoxidation und mit den resultierenden α,β-monoethylenisch ungesättigten Carbonsäuren chemisch verwandten Aldehyden besteht (so enthalten Acryl- und Methacryl-Rohsäure als Verunreinigungen neben Essig-, Ameisen- und Propionsäure in der Regel Formaldehyd, Acetaldehyd, Acrolein, Methacrolein, Propionaldehyd, n-Butyraldehyd, Benzaldehyd, Furfural und Crotonaldehyd).

Für verschiedene Anwendungen der α,β-monoethylenisch ungesättigten Carbonsäuren wirken sich die in den Rohsäuren enthaltenen Verunreinigungen nachteilig aus (vgl. z.B. DE-AS 22 07 184). So kann z.B. die Induktionszeit von Polymerisationsreaktionen, d.h. der Zeitraum zwischen dem Erreichen der Polymerisationstemperatur und dem tatsächlichen Beginn der Polymerisation, nicht reproduzierbar oder der Polymerisationsgrad reduziert sein. Auch können die Polymerisate zum Verfärben neigen.

Deshalb ist man für solche Anwendungszwecke bestrebt, die Verunreinigungen aus den Rohsäuren weitgehend abzutrennen und die Rohsäuren in Reinsäuren überzuführen. Dies kann z.B. durch zwei sich anschließende Rektifikationsstufen zur Entfernung leichter und schwerer als die Säure siedender Verunreinigungen erfolgen (vgl. z.B. EP-B 102 642).

Problematisch ist jedoch, daß wenigstens ein Teil der Verunreinigungen selbst bei behutsamer Vorgehensweise (z.B. Rektifikation unter reduziertem Druck, Zusatz von Stabilisatoren) diese weiteren Reinigungsprozesse der Rohsäuren dadurch behindert, daß er die Neigung zur Polymerisatbildung erhöht oder daß er in seinem physikalischen Verhalten der α,β-monoethylenisch ungesättigten Carbonsäure so ähnlich ist, daß eine rektifikative Entfernung derselben nur unter Anwendung einer unwirtschaftlichen Anzahl von Trennböden und/oder eines unwirtschaftlichen Rücklaufverhältnisses möglich ist und/oder aufgrund von Polymerisatbildung bereits nach kurzer Verfahrensdauer abgebrochen werden muß.

Aus der EP-B 102 642 ist daher bekannt, einen Teil der üblicherweise in der Rohsäure enthaltenen Verunreinigungen bereits auf dem Weg zur Methacryl-Rohsäure zu entfernen, indem die nach Absorption in Wasser anfallende wäßrige Methacrylsäurelösung mit einer wäßrigen Bisulfitlösung versetzt wird, bevor man aus der wäßrigen Lösung die Methacrylsäure durch Extraktion mit einem geeigneten organischen Lösungsmittel entfernt und anschließend das Lösungsmittel zur Herstellung der Rohsäure destillativ abtrennt.

Diese Verfahrensweise vermag jedoch nicht voll zu befriedigen und es wäre wünschenswert, ein effektives und einfaches Verfahren zur Verfügung zu haben, das die Rohsäure einer zusätzlichen Reinigungsstufe unterwirft.

Aus der DE-B 22 07 184 ist ein Verfahren zur Reinigung von Acryl-Rohsäure bekannt, das dadurch gekennzeichnet ist, daß man der Acryl-Rohsäure wenigstens eine der Verbindungen Hydrazin, Phenylhydrazin, Anilin, Monoethanolamin, Ethylendiamin, Glycin und Schwefelsäure zugibt und aus dem Gemisch die Acrylsäure unter vermindertem Druck destillativ abtrennt.

Nachteilig an diesem Verfahren ist jedoch, daß Amine in an sich bekannter Weise (vgl. z.B. EP-B 169 254) die Eigenschaft aufweisen, die Polymerisationsneigung α,β-monoethylenisch ungesättigter Carbonsäuren zu erhöhen und Hydrazine darüberhinaus hinsichtlich ihrer Handhabung problembehaftet sind. Eigene Untersuchungen haben ergeben, daß die Verwendung von Schwefelsäure insofern von Nachteil ist, als Schwefelsäure mit den Verunreinigungen in der Rohsäure schwerlösliche Verbindungen bildet, weshalb es zur Abscheidung von Feststoffen kommt.

Aus der EP-B 169 254 ist bekannt, α,β-monoethylenisch ungesättigte Rohcarbonsäuren mit einer Mercaptoverbindung in Gegenwart eines Säurekatalysators zu behandeln und die Säure destillativ abzutrennen. Als ein möglicher Säurekatalysator wird auch p-Toluolsulfonsäure genannt. Nachteilig an dieser Verfahrensweise ist jedoch der Bedarf der unangenehmen Mercaptoverbindungen.

Die JP-A 55 129239 beschreibt ein Verfahren zur Reinigung von Rohsäure α,β-monoethylenisch ungesättigter Carbonsäure, bei dem man die Rohsäure in Anwesenheit von Phenothiazin mit einer Arylsulfonsäure versetzt und aus dem Gemisch die ungesättigte Carbonsäure unter vermindertem Druck destillativ abtrennt. Die resultierende modifizierte Rohsäure wird als eine erhöhte Polymerisationsneigung aufweisend beschrieben.

Aufgabe der vorliegenden Erfindung war daher ein Verfahren zur Reinigung von Rohsäuren α,β-monoethylenisch ungesättigter Carbonsäuren, die nach dem Verfahren der katalytischen Gasphasenoxidation erzeugt wurden, das die genannten Nachteile nicht aufweist. Demgemäß wurde ein Verfahren zur Reinigung von Rohsäuren α,β-monoethylenisch ungesättigter Carbonsäuren, die nach dem Verfahren der katalytischen Gasphasenoxidation erzeugt wurden, gefunden, das dadurch gekennzeichnet ist, daß man die Rohsäure in Abwesenheit einer Mercaptoverbindung mit einer Arylsulfonsäure versetzt, aus dem Gemisch die α,β-monoethylenisch ungesättigte Carbonsäure unter vermindertem Druck destillativ abtrennt und die so erhältliche modifizierte Rohsäure einer α,β-monoethylenisch ungesättigten Carbonsäure nachfolgend rektifikativ zu einer Reinsäure einer α,β-monoethylenisch ungesättigten Carbonsäure aufarbeitet. Besonders geeignete Arylsulfonsäuren sind Toluolsulfonsäuren, Benzolsulfonsäure und Phenolsulfonsäuren, unter denen die p-Toluolsulfonsäure besonders bevorzugt ist.

In der Regel werden auf die Rohsäure bezogen 1 bis 5 Gew.-% Arylsulfonsäure zugesetzt und die destillative Abtrennung der α,β-monoethylenisch ungesättigten Carbonsäure bei einem Druck ≦ 100 mbar vorgenommen. Im Fall der Acryl- und Methacrylsäure liegt die zugehörige Siedetemperatur daher üblicherweise im Bereich von 70 bis 100°C. Bei Anwendung geringerer Mengen Arylsulfonsäure ist die reinigende Wirkung in der Regel nicht ausreichend und die Verwendung von mehr als 5 Gew.-% verbessert die Trennwirkung normalerweise nicht.

Besonders vorteilhaft wird das erfindungsgemäße Verfahren im Fall von 3 bis 6 C-Atome aufweisenden α,β-monoethylenisch ungesättigten Carbonsäuren, vorzugsweise im Fall von Acryl- und Methacrylsäure, angewendet, wobei seine Anwendung im Fall der Methacrylsäure ganz besonders vorteilhaft ist. Dies gilt vor allem dann, wenn die gasphasenkatalytische Herstellung im Fall der Acryl-Rohsäure ausgehend von Acrolein und im Fall der Methacryl-Rohsäure ausgehend von Methacrolein erfolgt, insbesondere dann, wenn das Acrolein selbst durch gasphasenkatalytische Oxidation von Propylen und das Methacrolein durch gasphasenkatalytische Oxidation von tert.-Butanol, iso-Butan oder iso-Buten oder durch Umsetzung von Formaldehyd mit Propionaldehyd gemäß EP-B 92 097 oder EP-B 58 927 erzeugt wird, und dieses insbesondere dann, wenn die gasphasenkatalytische Oxidation des Propylens, tert. Butanols, iso-Butans oder iso-Butens unter Verwendung einer katalytisch aktiven Masse der allgemeinen Formel I

Mo₁₂BiₐFe_{b}X¹_{c}X²_{d}X³ₑX⁴_{g}Oₙ (I),

in der die Variablen folgende Bedeutung haben:
- X¹: Nickel und/oder Kobalt,
- X²: Thallium, ein Alkalimetall und/oder ein Erdalkalimetall,
- X³: Phoshor, Arsen, Bor, Antimon, Zinn, Cer, Blei, Niob und/oder Wolfram,
- X⁴: Silicium, Aluminium, Titan und/oder Zirkonium,
- a: 0,5 bis 5,0
- b: 0,01 bis 3,0
- c: 3,0 bis 10,0
- d: 0,02 bis 2,0
- e: 0 bis 5,0
- f: 0 bis 10 und
- n: eine Zahl, die durch die Wertigkeit und Häufigkeit der von Sauerstoff verschiedenen Elemente in I bestimmt wird,
bei Temperaturen von 300 bis 400°C und, vom speziellen Temperaturverlauf abgesehen, ansonsten gemäß den Bedingungen der älteren deutschen Anmeldung P 40 23 239.5 (O.Z. 0050/41774) erfolgt und das anfallende Acrolein bzw. Methacrolein ohne Zwischenreinigung zur Weiteroxidation verwendet wird. Ferner bewährt sich das erfindungsgemäße Verfahren besonders dann, wenn die gasphasenkatalytische Oxidation des Acroleins bzw. Methacroleins, vom speziellen Temperaturverlauf abgesehen, gemäß den Ausführungen der älteren deutschen Anmeldung P 41 32 263.0 (O.Z. 0050/42719) bei Temperaturen von 200 bis 350°C bzw. gemäß der älteren deutschen Anmeldung P 41 32 684.9 (O.Z. 0050/42725) bei Temperaturen von 250 bis 400°C erfolgt.

Selbstverständlich erfolgt das erfindungsgemäße Verfahren in Gegenwart von Polymerisationsinhibitoren wie Luft, Hydrochinon, Hydrochinonmonoethylether oder Phenothiazin. Üblicherweise werden sie, bezogen auf die Rohsäure, in Mengen von 50 bis 1000 ppm eingesetzt.

Besonders vorteilhaft hat sich das erfindungsgemäße Verfahren in Gegenwart von aromatischen Hydroxyverbindungen wie Hydrochinon und Hydrochinonmonomethylether als Polymerisationsinitiatoren erwiesen.

Die nach dem erfindungsgemäßen Verfahren erhältlichen modifizierten Rohsäuren sind dadurch charakterisiert, daß ihre Neigung zur Polymerisatbildung, z.B. bei der Anwendung weiterer Trennverfahren, reduziert ist und daß sie im Vergleich zu den Rohsäuren insbesondere ärmer an solchen Verunreinigungen sind, die in ihrem physikalischen Verhalten der jeweiligen α,β-monoethylenisch ungesättigten Carbonsäure besonders ähnlich sind, so daß eine rektifikative Aufarbeitung zur Reinsäure wesentlich vereinfacht wird. Die Verarmung der modifizierten Rohsäure an Verunreinigungen weist sich z.B. durch eine Schwarzfärbung der Arylsulfonsäure aus, die die Aufnahme der Verunreinigungen in der Arylsulfonsäure anzeigt. Bemerkenswerterweise erfolgt die Aufnahme der Verunreinigungen in die Arylsulfonsäuren ohne Abscheidung von Feststoffen, weshalb sich das erfindungsgemäße Verfahren insbesondere für eine kontinuierliche Ausführungsweise eignet, bei der man die Rohsäure in einfacher Weise kontinuierlich einem Gemisch aus α,β-monoethylenisch ungesättigter Carbonsäure und Arylsulfonsäure zuführt und aus diesem unter reduziertem Druck die α,β-monoethylenisch ungesättigte Carbonsäure kontinuierlich abdestilliert, wobei von Zeit zu Zeit verbrauchte Arylsulfonsäure entfernt und durch frische Arylsulfonsäure ersetzt wird. In der Regel wird dabei mit mittleren Verweilzeiten der α,β-monoethylenisch ungesättigten Carbonsäuren von 10 bis 60 min gearbeitet.

### Beispiel

Gemäß Beispiel 1 der EP-B 58 927 wurde durch Kondensation von Formaldehyd und Propionaldehyd und anschließende azeotrope Destillation Methacrolein hergestellt. Aus dieser wurde gemäß dem Ausführungsbeispiel der EP-A 297 445 (katalytische Gasphasenoxidation) eine wäßrige Methacrylsäurelösung gewonnen. Dies wurde mit der für die Oxidation eingesetzten Syntheseluft (Frischluft) bei 50 bis 70°C gestrippt (Frischluftmenge siehe Tabelle 1 der EP-A 297 445), danach gemäß Beispiel 6 der Patentschrift 54 354 der Deutschen Demokratischen Republik mit Isooktansäure extrahiert und das dabei anfallende Methacrylsäure/Isooktansäure-Gemisch gemäß Beispiel 10 derselben Schrift destillativ aufgetrennt. Die so erhaltene Methacryl-Rohsäure wurde, bezogen auf die Methacryl-Rohsäure, mit 3 Gew.-% p-Toluolsulfonsäure versetzt und von diesem Gemisch bei einem Druck von 100 mbar und einer Temperatur von 100°C Methacryl-Rohsäure destillativ abgetrennt. Anschließend wurde die so erzeugte Rohsäure kontinuierlich (0,5 l/h) in eine Rektifikationskolonne für niedrigsiedende Verunreinigungen eingespeist, die Niedrigsieder über Kopf abgetrennt, der Sumpfaustrag kontinuierlich in eine Destillationsblase überführt und die Methacrylsäure über Kopf in hoher Reinheit gewonnen (Sumpftemperatur: 105°C, Druck: 100 mbar).

Es handelte es sich um eine isolierte, doppelwandig verspiegelte Dual-Flow-Bodenkolonne (Durchmesser: 50 mm, Länge: 1 m, Bodenzahl: 20, Bodentyp: Dual-Flow, Sumpftemperatur: 100°C, Druck: 100 mbar, Rücklaufverhältnis: 1). Nach einer kontinuierlichen Betriebsdauer von 1 Woche war noch keine Polymerisatabscheidung auf den Böden festzustellen.

Unter gleichen Bedingungen wurde das Beispiel wiederholt, der erfindungsgemäße Reinigungsschritt mit p-Toluolsulfonsäure jedoch unterlassen. Als Ergebnis mußte die rektifikative Reinigung der Rohsäure nach wenigen Stunden aufgrund von Polymerisatbildung abgebrochen werden und die gewonnene Reinsäure enthielt einen vergleichweise erhöhten Gehalt an Verunreinigungen. In beiden Fällen wurde in Anwesenheit von 100 ppm (bezogen auf das Gewicht der Methacrylsäure) Hydrochinonmonomethylether gearbeitet.

## Patentansprüche

1. Verfahren zur Reinigung von Rohsäuren α,β-monoethylenisch ungesättigter Carbonsäuren, die nach dem Verfahren der katalytischen Gasphasenoxidation erzeugt wurden, dadurch gekennzeichnet, daß man die Rohsäure in Abwesenheit einer Mercaptoverbindung mit einer Arylsulfonsäure versetzt, aus dem Gemisch die α,β-monoethylenisch ungesättigte Carbonsäure unter vermindertem Druck destillativ abtrennt und die so erhältliche modifizierte Rohsäure einer α,β-monoethylenisch ungesättigter Carbonsäure nachfolgend rektifikativ zu einer Reinsäure einer α,β-monoethylenisch ungesättigten Carbonsäure aufarbeitet.

2. Verfahren nach Anspruch 1, dadurch gekennzeichnet, daß die α,β-monoethylenisch ungesättigte Carbonsäure Acryl- oder Methacrylsäure ist.

3. Verfahren nach den Ansprüchen 1 oder 2, dadurch gekennzeichnet, daß die α,β-monoethylenisch ungesättigte Carbonsäure Methacrylsäure ist.

4. Verfahren nach den Ansprüchen 1 bis 3, dadurch gekennzeichnet, daß die Arylsulfonsäure p-Toluolsulfonsäure ist.

5. Verfahren nach den Ansprüchen 1 bis 4, dadurch gekennzeichnet, daß es kontinuierlich ausgeführt wird.

## Claims

1. A process for the purification of crude α,β-monoethylenically unsaturated carboxylic acids produced by catalytic gas-phase oxidation, which comprises treating the crude acid with an arylsulfonic acid in the absence of a mercapto compound, removing the α,β-monoethylenically unsaturated carboxylic acid from the mixture by distillation under reduced pressure and subsequently working up by rectification the modified crude acid of an α,β-monoethylenically unsaturated carboxylic acid which is obtainable in this way, to give a pure acid of an α,β-monoethylenically unsaturated carboxylic acid.

2. A process as claimed in claim 1, wherein the α,β-monoethylenically unsaturated carboxylic acid is acrylic acid or methacrylic acid.

3. A process as claimed in claim 1 or 2, wherein the α,β-monoethylenically unsaturated carboxylic acid is methacrylic acid.

4. A process as claimed in claim 1 or 2 or 3, wherein the arylsulfonic acid is p-toluenesulfonic acid.

5. A process as claimed in claim 1 or 2 or 3 or 4, which is carried out continuously.

## Revendications

1. Procédé de purification d'acides carboxyliques à insaturation α,β-monoéthylénique bruts, qui ont été produits par le procédé d'oxydation catalytique en phase gazeuse, caractérisé en ce que l'on additionne l'acide brut d'un acide arylsulfonique en l'absence d'un composé mercapto, on sépare du mélange, par distillation sous pression réduite, l'acide carboxylique à insaturation α,β-monoéthylénique et on soumet ensuite l'acide carboxylique à insaturation α,β-monoéthylénique brut modifié ainsi obtenu à un traitement ultérieur de rectification pour obtenir un acide carboxylique à insaturation α,β-monoéthylénique pur.

2. Procédé selon la revendication 1, caractérisé en ce que l'acide carboxylique à insaturation α,β-monoéthylénique est l'acide acrylique ou méthacrylique.

3. Procédé selon la revendication 1 ou 2, caractérisé en ce que l'acide carboxylique à insaturation α,β-monoéthylénique est l'acide méthacrylique.

4. Procédé selon l'une quelconque des revendications 1 à 3, caractérisé en ce que l'acide arylsulfonique est l'acide p-toluènesulfonique.

5. Procédé selon l'une quelconque des revendications 1 à 4, caractérisé en ce qu'il est exécuté en continu.
